# EUROPEAN PATENT SPECIFICATION

(11) **EP 2 396 040 B1**
(45) Date of publication and mention of the grant of the patent: **05.04.2017**
(21) Application number: 10703282.3
(22) Date of filing: 11.02.2010
(51) Int. Cl.: A61K 51/00, A61K 103/34, A61P 43/00, C07F 5/00

(54) **USE OF BUFFERS FOR RADIONUCLIDE COMPLEXATION**
VERWENDUNG VON PUFFERN FÜR RADIONUKLEID-KOMPLEXIERUNG
UTILISATION DE TAMPONS POUR LA COMPLEXATION DE RADIONUCLÉIDES

(30) Priority: 13.02.2009 FR 0950944; 23.02.2009 US 154650 P
(43) Date of publication of application: 21.12.2011
(73) Proprietor: GUERBET, 93420 Villepinte (FR)
(72) Inventor: PORT, Marc, F-95170 Deuil La Barre (FR); MEDINA, Christelle, F-77360 Vaires sur Marne (FR)
(74) Representative: Regimbeau
(86) International application number: PCT/EP2010/051709
(87) International publication number: WO 2010/092114

(56) References cited:
- WO-A-2004/089425
- WO-A-2007/042504
- VELIKYAN I ET AL: "Convenient preparation of <68>Ga-based PET-radiopharmaceuticals at room temperature" BIOCONJUGATE CHEMISTRY 200802 US, vol. 19, no. 2, February 2008 (2008-02), pages 569-573, XP002546370 ISSN: 1043-1802 cited in the application

## Description

The invention relates to improved compositions of contrast agents and to a method for preparing such compositions. The invention relates in particular to products for PET (positron emission tomography) imaging, and especially products containing a radionuclide for PET imaging, gallium 68. Document WO 2007/042504 in the name of the applicant explains in detail the advantage of PET imaging, in particular with gallium 68 (Ga68) which is very advantageous since it does not require the use, in the vicinity of or within the hospital, of a very bulky and expensive cyclotron. The Ga68 produced by a small dedicated generator is coupled to a "vectorized chelate" (referred to without distinction as "cold kit" in the present application) which is not yet labeled, the coordination reaction (complexing of the radionuclide by the cold kit) providing a Ga68-"radiolabeled vectorized chelate" (referred to without distinction as "complexed vectorized chelate" in the present application), which is administered to the patient for PET imaging.

More specifically, very advantageously, the vectorized chelate comprises "a targeting part" (referred to without distinction as "targeting agent" or "biovector" in the present application) for targeting a specific biological target (cell receptor, for example), in particular a pathological region of diagnostic interest, said targeting part being linked, typically by a chemical linker (covalent bond via any appropriate bonding group) to the chelate constituting the signal part. This chelate is capable of complexing the radionuclide (for example, a macrocyclic chelate such as DOTA, NOTA or PCTA).

More specifically, the solution of radioactive Ga68, the gallium-generator eluate, is mixed, in a dedicated and protected area of the hospital (area denoted radiopharmacy), with a vectorized-chelate solution prepared in advance (the cold kit), provided by the manufacturer of the contrast product.

Advantageously, the mixing is carried out in an automated device into which are introduced, on the one hand, the dose of generator-eluate Ga68 and, on the other hand, the dose of vectorized chelate (not yet radiolabeled with Ga68). Given the short half-life of the radionuclide (68 minutes for gallium 68) in clinical practice, the intention is always to improve the conditions for preparing the radiolabeled contrast agent, and in particular to obtain a very rapid and simple complexation with a high degree of purity of the radiolabeled chelate. More specifically, the intention is to obtain a complexation in a very short time, with a yield and a purity for the radiolabeled chelate of at least 90%, preferably of at least 95%.

For gallium technology, the prior art first of all described gallium complexation carried out by heating at a temperature of the order of 75°C to 95°C, for at least 15 minutes. A satisfactory degree of purity was achieved at these temperatures using HEPES (sulfonic acid derivative) or acetate buffers. However, this heating time is, on the other hand, relatively long compared with the short life of the Gallium68 isotope, and requires appropriate equipment.

These buffers were, to the applicant's knowledge, up until now recognized as the only ones used by those skilled in the art of gallium complexation, probably owing to their proven effectiveness under the physicochemical conditions desired for this complexation, in particular the obtaining of a pH between 3.5 and 4.5, and to their common use in PET-compound biochemistry.

However, these buffers have the following drawbacks:
- their use requires very specific physicochemical conditions (in particular of concentration and of pH). Thus, they do not make it possible to obtain a broad working pH range for the complexation, which is very limiting for the user. In particular, this fixes the working conditions, with risks of non-compliance with the regulations, which is of course a major problem for the pharmaceutical product. For example, if the solution of Ga68 eluate derived from the generator is more acidic, the buffer used may no longer have the required buffering capacity, which may disrupt the complexation and/or impair the biovector, and thus mean that the product does not comply with the specifications that it has to meet in order to be injected into the patient;
- the HEPES buffer does not appear in the pharmacopea, thereby posing complex pharmaceutical regulation problems in terms of clinical use in humans.

In order to improve the complexation process, the prior-art research did not relate to the buffer solutions that can be used, but to the method of heating or the need for such heating.

Thus, the prior art subsequently described, in document WO2004/089425 a more rapid microwave method of coordination intended to avoid the heating step while at the same time still using the same buffers. However, this microwave step also leads to constraints for the user, and represents a risk of impairment and/or of degradation of the vectorized chelate, pharmacological biovectors in fact often being sensitive to this treatment, in particular peptides, organic pharmacophores, vitamins and proteins.

In order to avoid these difficulties, the publication Velikyan et al, Bioconjugate Chem, 2008, 19, 569-573 describes a method of coordination at ambient temperature and without microwaves, but using the buffers already used previously, more exactly HEPES or sodium acetate buffers, at certain very specific concentration and pH values. More specifically, the HEPES buffer is described as effective at a pH of 4.6-4.8 and 1 M, and the acetate buffer is described as effective at a pH of 4.6-4.8 and 0.4 M.

Thus, overall, the prior art teaches that complexation, in particular at ambient temperature, requires the use only of certain buffers (HEPES, acetate) and under very specific physicochemical conditions.

Thus, in view of the prior art, only certain very particular, quite strict physicochemical conditions enable the use of these buffers at ambient temperature. The invention aims to solve the problem of obtaining buffers that are efficient for gallium complexation, and that can be used at various chemical conditions.

Surprisingly, the applicant has noted that complexation, in particular at ambient temperature, with neither a heating step nor microwaves, is obtained successfully, in particular in terms of the rapidity of complexation (in less than 15 minutes, preferably in less than 10 minutes, more preferably in 3 to 10 minutes, for example 5 to 8 minutes) and of the quality of the radiolabeled chelates, through the use, for the complexation reaction, of certain appropriate buffers of the pharmacopea other than HEPES and acetate, under physicochemical complexation conditions (in particular concentrations and pH) that are less strict and limiting than in the prior art. Very satisfactory results have in particular been obtained in the case of NOTA and PCTA, and in the case of several buffers which have been observed, by the applicant, to have comparable physicochemical characteristics. It has even been possible to obtain these results at ambient temperature, without heating or microwave, which is particularly surprising in view of the prior art. It is specified that *a fortiori,* since these buffers can be used at ambient temperature, the pharmacopea buffers of the applicant can be used with heating or microwaves, which may be desired for certain biovectors for example (or if the automated device which performs the complexation includes this programming automatically).

The buffers of the applicant enable satisfactory complexation, with a suitable degree of complexation, advantageously at least 92%, 95%, 97%, and a sufficient purity (at least 92%, 95%, 97%) and preferably without the formation of precipitate to be eliminated.

More specifically, the applicant has obtained very advantageous results with the buffers comprising at least two Gallium 68-coordination functions described below and as described in the detailed examples.

To this effect, according to a first aspect, the invention relates to a method for complexation (referred to without distinction as "method of radiolabeling" in the present application) of a chelate selected from NOTA, PCTA, vectorised NOTA or vectorised PCTA with a radionuclide, gallium, the complexation being carried out, at ambient temperature without heating, by adding the radionuclide to the chelate in a buffer solution, the buffer of this solution comprising between two and five functions for coordination with the radionuclide, each coordination function being independently chosen from a carboxylic acid function and a hydroxyl function, on the condition that the buffer comprises at least one carboxylic acid function and at most two carboxylic acid functions. Said buffer is a C₁-C₁₀ monocarboxylic acid which is monohydroxylated, optionally di-, tri- or tetrahydroxylated, and optionally unsaturated, or a C₁-C₁₀ dicarboxylic acid which is optionally mono-, di- or trihydroxylated, and optionally unsaturated.

Thus, the applicant has made a judicious choice of the buffers in such a way that the complexation kinetics are sufficiently rapid to allow varied and flexible conditions for use (in particular pH and heating conditions).

The function sulfonic acid is excluded from the definition of carboxylic acid function.

In the present application, the buffers can carry the hydroxyl function directly on the carbon of the C=O group of the carboxylic acid function (as in the case of the carbonate buffer), or else on another carbon of the carbon chain (hence alpha, beta, etc., omega hydroxy carboxylic acids).

The invention covers the case of a poly acid/base function having several pKa of between 2 and 13, associated with the same functional group (the case of the phosphoric acid buffer in particular).

Moreover, the complexation is even better when, in the case where the buffer contains two carboxylic acid functions, it also contains at least one hydroxyl function (better complexation without the need to filter off precipitate after the complexation). Thus, preferably, the invention relates to a method of radio labeling characterized in that the buffer also comprises a hydroxyl function.

According to embodiments, the buffers used advantageously comprise two carboxylic acid functions and at least one hydroxyl function, and in particular one or two hydroxyl functions.

According to embodiments, the buffers used advantageously comprise at least one carboxylic acid function and at least one hydroxyl function. Advantageously, the buffer is chosen from the following buffers: lactate, tartrate, malate, maleate, succinate and carbonate, and mixtures thereof.

The buffer is a C₁-C₁₀ monocarboxylic acid which is monohydroxylated, optionally di-, tri- or tetrahydroxylated, and optionally unsaturated (in particular having at least one double bond), or a C₁-C₁₀ dicarboxylic acid which is optionally mono-, di- or trihydroxylated, and optionally unsaturated (in particular having at least one double bond).

These buffers so belong very advantageously to the pharmacopea.

Very advantageously, the buffer is chosen from the following buffers: lactate, tartrate and malate, or mixtures thereof.

It is recalled here that:
- tartrate has two carboxylic acid functions and two hydroxyl functions;
- lactate has one carboxylic acid function and one hydroxyl function.

It is recalled that HEPES and acetic acid comprise, respectively, zero and a single carboxylic acid function.

**Table 1 recalls the formulae of the buffers which are not comprised within the claimed invention.**

| **Buffer** | **Formula** | pKa |
|---|---|---|
| citrate (three carboxylic acid functions) | 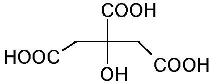 | 3.15; 4.77 and 6.4 |
| Acetate (one carboxylic acid function) | 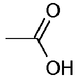 | 4.75 |
| HEPES (one sulfonic acid function) | 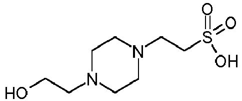 | 3 and 7.55 |

**Table 2 recalls the formulae of the buffers according to the present invention**

| **Buffer** | **Formula** | pKa |
|---|---|---|
| lactate | 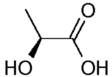 | 3.8 |
| tartrate | 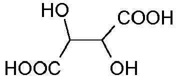 | 3.04 and 4.37 |
| malate | 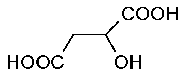 | 3.46 and 5.10 |
| | | |
| carbonate | 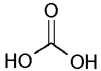 | 6.35 and 10.33 |
| succinate | 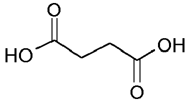 | 4,2 and 6,1 |
| maleate | 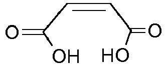 | 1,8 and 6,1 |

The pH of the solution for complexation using the buffers of the invention is advantageously between 1 and 14, typically between 3 and 11, advantageously between 3 and 7.

More specifically, it is recalled that the buffering capacity is the pKa value +/- 1, which means, for example, 3.75-5.75 for acetate. By virtue of the buffers of the applicant, the range is much broader, for example [2.04-5.37] for tartrate, [5.35-11.33] for carbonate and [1.12-13.67] for phosphate. It is also possible to combine several buffers in combination (buffer mixtures) in particular so as to optimize the buffering capacity ranges and the choice of the chelates and of the biovectors, it being specified that not all chelates necessarily have the same complexation, depending on the buffer under consideration. Thus, for example, NOTA is particularly advantageous for lactate, carbonate or tartrate buffers.

The exact mechanisms are unknown, but the applicant puts forward the hyopothesis, in order to explain these results *a posteriori,* that the very effective buffers are those which contain:
- a sufficient number of coordination functions to sufficiently complex gallium: acetate, which contains just one function, would not be sufficiently complexing to be effective for varied complexation conditions (concentration, pH); HEPES which contains one sulfonate function and one hydroxyl group, would also not be sufficiently complexing;
- but not too many coordination functions, so as not to complex gallium too much: citrate, which contains three carboxylic acid functions, is found to be too complexing.

By extension, the invention concerns the case of buffers such that the log K Ga68 value is between 3 and 6, preferably between 3 and 5.

The applicant has realized that the constant for Ga68 complexation by the buffer (log K Ga68 of the buffer comprising between 2 and 4 coordination functions) is very advantageously between 2 and 6, and very advantageously between 3 and 5, preferably of the order of 4 (between 3.5 and 4.5 in particular).

In a certain way, the applicant has gone against a prejudice to use known organic compounds such as lactate as gallium-complexing agents, these compounds being used in therapy as anticancer medicaments or as tumor-imaging agents (such compounds could have lead to an interference towards the chelate for the gallium complexation).

According to embodiments, the buffer is a combination of at least two of the buffers indicated above, for example a mixture of lactate and tartrate buffer. All combinations are possible with, for example, the following proportions between the buffers of the present invention: 90/10, 80/20, 60/40, 50/50 for two buffers, and 80/10/10, 60/20/20 for three buffers. A mixture, for example, of a lactate-type buffer according to the invention very effective for a given chelate and a buffer less effective for this chelate could also be used. It is also possible to use, less advantageously, a mixture of very effective buffer according to the invention (lactate, for example), with a buffer that is not effective alone (citrate, in particular), but on the condition of using a sufficiently small proportion of the buffer that is not effective alone so as not to impair the complexation.

The chelate that can be used in the context of the present invention is a free (i.e. nonvectorized) chelate or a vectorized chelate, the chelate being capable of complexing the radionuclide. Advantageously, it is a vectorized chelate. For the purpose of the present invention, the term "vectorized chelate" is intended to mean any chelate to which is bound (by means of a chemical linker) a part for targeting a specific biological target, in particular a region of diagnostic interest. Thus, advantageously, the chelate is vectorized with an agent for targeting a specific biological target, in particular a region of diagnostic interest.

Advantageously, the chelate part of the vectorized chelate is well known to those skilled in the art, and it is preferably one of the chelates preferred for the complexation of Ga68, such as NOTA and its derivatives, PCTA and its derivatives.

Thus, the vectorized chelate is advantageously chosen from vectorized NOTA, vectorized PCTA, advantageously dideaza-NOTA, dideaza PCTA, folate NOTA, folate PCTA.

The applicant has obtained particularly advantageous results for:
- a lactic, tartaric or carbonic buffer for NOTA;
- a lactic or carbonic buffer for PCTA.

For the chelate part of the product, a large number of chelates can be used, in particular
a macrocyclic chelate having the general formula below: 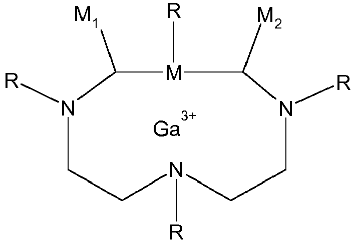 where:
M-M1-M2 forms a pyridine nucleus,
or M1 and M2 are absent and M represents a bond,
with at least one R being CHXCO₂- and X being L(Linker)-B (Biovector);
Use may in particular be made of a 3,6,9,15-tetraazabicyclo[9.3.1]pentadeca-1(15),11,13-triene-3,6,9-triacetic acid (PCTA).

Use may also be made of derivatives in which one or more carboxylic groups are in the form of a corresponding salt, ester or amide.

More broadly, the chelate(s) forming the signal entity may correspond to the formula of document WO2007/042504 (incorporated by way of reference for the formulae and the associated definitions), and of document WO01/60416, which follow: 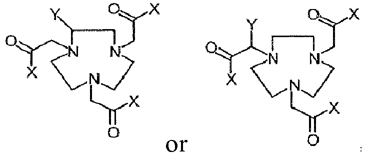 with X a group capable of coordinating a metal cation (preferably 0-, OH, NH₂, OPO₃-, NHR with R an aliphatic chain), and Y a linking group capable of being linked to a biovector (for instance Y is (CH2)ₙ - CO₂H with n being 1 to 5, advantageously n=2).

Use may advantageously be made of NOTA compounds which are denoted C-functionalized (at least one group Y grafted onto a carbon atom of the NOTA enables the coupling of the biovector) and NOTA compounds carrying at least one additional CH₂ group in the ring. Advantageously, it is the NOTA derivative of formula

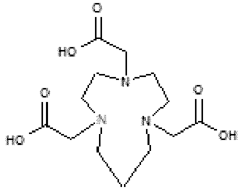

Use may also be made of the chelates with a PCTA backbone, described by the applicant especially in US 6 440 956.

Use may also be made of NOTA derivatives.

Use may also be made of the following chelates:

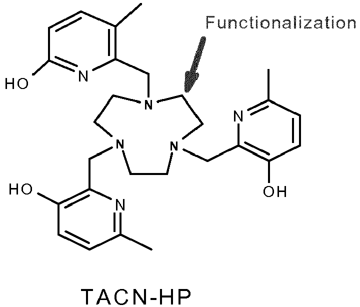

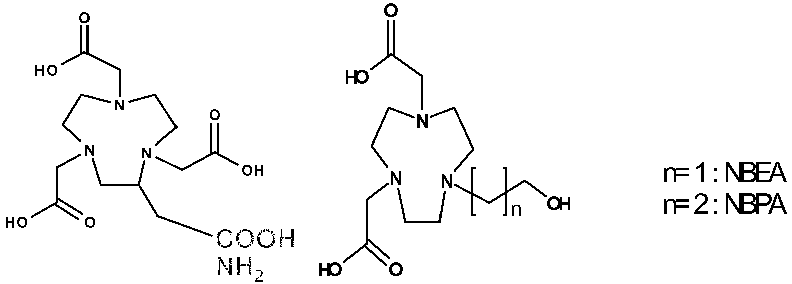

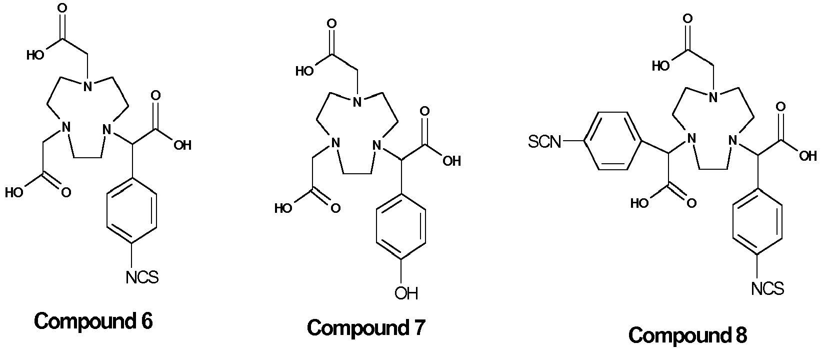

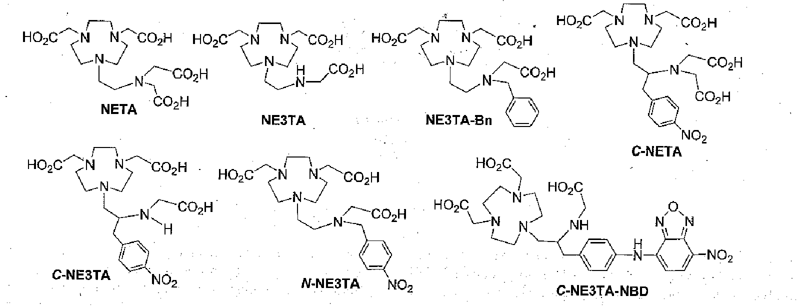

Advantageously, the vectorized-chelate-targeting part is a biovector for targeting a pathological region of diagnostic interest, the biovector being advantageously an amino acid, a peptide, advantageously comprising 4 to 15, or 4 to 10 amino acids, a polypeptide, a vitamin, a monosaccharide or polysaccharide, an antibody, a nucleic acid, a bicyclam or an aptamer. It may also be a biovector targeting cell receptors (in particular all the receptors described below), a pharmacophore (organic molecule with pharmacological activity), an angiogenesis-targeting biovector, an MMP-targeting biovector, a tyrosine-kinase-targeting peptide, an atheroma-plaque-targeting peptide or an amyloid-plaque-targeting biovector, a VCAM targeting biovector (peptidic or non peptidic).

More broadly, the biovector(s) is (are), for example, chosen from the following list (the documents and references between parentheses are examples and not a limiting list):
1) Biovectors targeting angiopoietin and VEGF receptors (described in WO 01/97850), polymers such as polyhistidine (US 6,372,194), fibrin-targeting polypeptides (WO 2001/9188), integrin-targeting peptides (WO 01/77145, WO 02/26776 for αvβ3, WO 02/081497, for example RGDWXE), pseudopeptides and peptides for targeting metalloproteases MMP (WO 03/062198, WO 01/60416), peptides targeting, for example, the KDR/Flk-1 receptor or Tie-1 and 2 receptors (WO 99/40947, for example), sialyl Lewis glycosides (WO 02/062810 and Muller et al, Eur. J. Org. Chem, 2002, 3966-3973), antioxidants such as ascorbic acid (WO 02/40060), tuftsin-targeting biovectors (for example, US 6,524,554), biovectors for targeting G protein receptors, GPCRs, in particular cholecystokinin (WO 02/094873), associations between an integrin antagonist and a guanidine mimic (US 6 489 333), αvβ3-targeting or αvβ5-targeting quinolones (US 6,511,648), benzodiazepines and analogs targeting integrins (US A 2002/0106325, WO 01/97861), imidazoles and analogs (WO 01/98294), RGD peptides (WO 01/10450), antibodies or antibody fragments (FGF, TGFβ, GV39, GV97, ELAM, VCAM, which are TNF- or IL-inducible (US 6,261,535)), targeting molecules which are modified by interaction with the target (US 5,707,605), agents for targeting amyloid deposits (WO 02/28441, for example), cleaved cathepsin peptides (WO 02/056670), mitoxantrones or quinones (US 6,410,695), epithelial-cell-targeting polypeptides (US 6,391,280), cysteine protease inhibitors (WO 99/54317), the biovectors described in: US 6,491,893 (GCSF), US 2002/0128553, WO 02/054088, WO 02/32292, WO 02/38546, WO 20036059, US 6,534,038, WO 0177102, EP 1 121 377, Pharmacological Reviews (52, No. 2, 179: growth factors PDGF, EGF, FGF, etc.), Topics in Current Chemistry (222, W.Krause, Springer), Bioorganic & Medicinal Chemistry (11, 2003, 1319-1341; αvβ3-targeting tetrahydrobenzazepinone derivatives).
2) Angiogenesis inhibitors, in particular those tested in clinical trials or already marketed, in particular:
   - inhibitors of angiogenesis involving FGFR or VEGFR receptors, such as SU101, SU5416, SU6668, ZD4190, PTK787, ZK225846, azacyclic compounds (WO 00244156, WO 02059110);
   - inhibitors of angiogenesis involving MMPs, such as BB25-16 (marimastat), AG3340 (prinomastat), solimastat, BAY12-9566, BMS275291, metastat, neovastat;
   - inhibitors of angiogenesis involving integrins, such as SM256, SG545, adhesion molecules which block EC-ECM (such as EMD 121-974, or vitaxin);
   - medicaments with a more indirect mechanism of antiangiogenic action, such as carboxyamidotriazole, TNP470, squalamine, ZD0101;
   - the inhibitors described in document WO 99/40947, monoclonal antibodies which are very selective for binding to the KDR receptor, somatostatin analogs (WO 94/00489), selectin-binding peptides (WO 94/05269), growth factors (VEGF, EGF, PDGF, TNF, MCSF, interleukins); VEGF-targeting biovectors described in Nuclear Medicine Communications, 1999, 20;
   - the inhibitory peptides of document WO 02/066512.
3) Biovectors capable of targeting receptors: CD36, EPAS-1, ARNT, NHE3, Tie-1, 1/KDR, Flt-1, Tek, neuropilin-1, endoglin, pleiotropin, endosialin, Axl., alPi, a2ssl, a4P1, a5pl, eph B4 (ephrin), the laminin A receptor, the neutrophilin receptor 65, the leptin receptor OB-RP, the chemokine receptor CXCR-4 (and other receptors mentioned in document WO 99/40947), bombesin/GRP, receptors for gastrin, VIP, CCK.
4) Biovectors of tyrosine kinase inhibitor type.
5) Known GPIIb/IIIa receptor inhibitors, chosen from: (1) the fab fragment of a monoclonal antibody against the GPIIb/IIIa receptor, Abciximab, (2) small peptide and peptidomimetic molecules injected intravenously, such as eptifibatide and tirofiban.
6) Fibrinogen receptor antagonist peptides (EP 425 212), IIb/IIIa receptor ligand peptides, fibrinogen ligands, thrombin ligands, peptides capable of targeting atheroma plaque, platelets, fibrin, hirudin-based peptides, guanine-based derivatives targeting the IIb/IIIa receptor.
7) Other biovectors or biologically active fragments of biovectors known to those skilled in the art as medicaments, having an anti-thrombotic, anti-platelet-aggregation, anti-atherosclerotic, anti-restenoic or anticoagulant action.
8) Other biovectors or biologically active fragments of biovectors targeting αvβ3, described in association with DOTAs in patent US 6 537 520, chosen from the following: mitomycin, tretinoin, ribomustin, gemcitabine, vincristine, etoposide, cladribine, mitobronitol, methotrexate, doxorubicin, carboquone, pentostatin, nitracrine, zinostatin, cetrorelix, letrozole, raltitrexed, daunorubicin, fadrozole, fotemustine, thymalfasin, sobuzoxane, nedaplatin, cytarabine, bicalutamide, vinorelbine, vesnarinone, aminoglutethimide, amsacrine, proglumide, elliptinium acetate, ketanserin, doxifluridine, etretinate, isotretinoin, streptozocin, nimustine, vindesine, flutamide, drogenil, butocin, carmofur, razoxane, sizofilan, carboplatin, mitolactol, tegafur, ifosfamide, prednimustine, picibanil, levamisole, teniposide, improsulfan, enocitabine, lisuride, oxymetholone, tamoxifen, progesterone, mepitiostane, epitiostanol, formestane, interferon-alpha, interferon-2 alpha, interferon-beta, interferon-gamma, colony stimulating factor-1, colony stimulating factor-2, denileukin diftitox, interleukin-2, leutinizing hormone releasing factor.
9) Certain biovectors targeting particular types of cancers, for example peptides targeting the ST receptor associated with colorectal cancer, or the tachykinin receptor.
10) Biovectors using phosphine-type compounds.
11) The biovectors for targeting P-selectin, E-selectin; for example, the 8-amino-acid peptide described by Morikawa et al, 1996, 951, and also various sugars.
12) Annexin V or biovectors targeting apoptotic processes.
13) Any peptide obtained by targeting technologies, such as phage display, optionally modified with unnatural amino acids (http//chemlibrary.bri.nrc.ca), for example peptides derived from phage display libraries: RGD, NGR, KGD, RGD-4C.
14) Other peptide biovectors known for targeting atheroma plaques, mentioned in particular in document WO 2003/014145.
15) Vitamins, in particular folic acid and its known derivatives capable of targeting folate receptors.
16) Ligands for hormone receptors, including hormones and steroids.
17) Opioid-receptor-targeting biovectors.
18) Biovectors targeting kinases, for example tyrosine kinases.
19) LB4 and VnR antagonists.
20) Nitroimidazole and benzylguanidine compounds.
21) Biovectors summarized in Topics in Current Chemistry, vol.222, 260-274, Fundamentals of Receptor-based Diagnostic Metallopharmaceuticals, in particular:
   - biovectors for targeting peptide receptors overexpressed in tumors (LHRH receptors, bombesin/GRP, VIP receptors, CCK receptors, tachykinin receptors, for example), in particular analogs of somatostatin or of bombesin, optionally glycosylated octreotide peptide derivatives, VIP peptides, alpha-MSHs, CCK-B peptides;
   - peptides chosen from: RGD cyclic peptides, fibrin-targeting peptides, tuftsin-targeting peptides, fMLF peptides (receptor: laminin).
22) Oligosaccharides, polysaccharides and derivatives of monosaccharides, derivatives targeting Glut receptors (monosaccharide receptors) or glutamine transporters.
23) Biovectors used for smart-type products.
24) Myocardial viability markers (for example, tetrofosmin and hexakis(2-methoxy-2-methylpropylisonitrile)).
25) Sugar and fat metabolism traces.
26) Ligands of neurotransmitter receptors (D, 5HT, Ach, GABA, NA, NMDA receptors).
27) Oligonucleotides and aptamers.
28) Tissue factor.
29) Biovectors described in WO 03/20701, in particular the PK11195 ligand for the peripheral benzodiazepine receptor.
30) Fibrin-binding peptides, in particular the peptide sequences described in WO 03/11115.
31) Amyloid plaque aggregation inhibitors described in WO 02/085903, for example.
32) Compounds for targeting Alzheimer's disease, in particular compounds comprising backbones of benzothiazole, benzofuran, styrylbenzoxazole/thiazole/imidazole/quinoline, styrylpyridine type, and known derivatives thereof.
33) Agents for targeting chemokine receptors, and in particular for targeting CXCR4.

Any RGD peptide is in particular interestingly used.

In one particular embodiment, the biovector is an agent for targeting chemokine receptors, and in particular for targeting CXCR4.

For targeting CXCR4, use will in particular advantageously be made of bicyclams (for example, all the derivatives described in WO2006032704) and peptides (in particular cyclic peptides) in particular peptides of 3 to 10 amino acids, preferably 3 to 8 amino acids, for example 4 to 6 amino acids. Advantageously, use will be made of a peptide among the following:
a) Cyclo(D-Tyr-X-Arg-Nal-Gly)

| **X** | **IC₅₀ (nM)** |
|---|---|
| Trans-4-guanidinoPro | 10 |
| Trans-4-guanidinoPro | 9.9 |
| Orn | 19 |
| Lys | 97 |
| gDab | 24 |
| gLys | 33 |

b) Cyclo(D-Tyr-Orn-Arg-Nal-Gly)
c) Cyclo(D-Tyr-Arg-Arg-X-Gly)

| **X** | **IC₅₀ (nM)** |
|---|---|
| Trp | 13 |
| Bth | 18 |

With Bth: 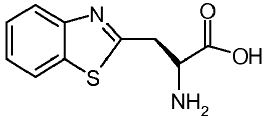
d) Cyclo(D-Tyr-Arg-Arg-Nal-D-Ala): IC₅₀ = 11nM
e) Cyclo(D-Tyr-Arg-Arg-Nal-D-Asp), Cyclo(D-Tyr-Arg-Arg-Nal-D-Glu), Cyclo(D-Tyr-Arg-Arg-Nal-D-Lys), Cyclo(D-Tyr-Arg-Arg-Nal-Gly)

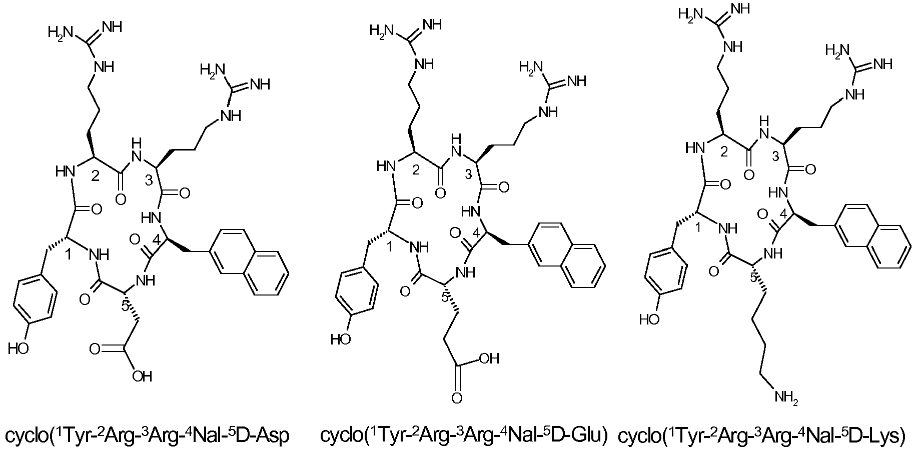

Compounds a) to e) used as biovectors, and coupled to the chelate complexing gallium, are not known form the prior art, either within the buffer solution of the present invention, or with the other buffers (acetate in particular) of the prior art. Use may be made of any known compound of pseudopeptide or nonpeptide type for targeting CXCR4, for example:

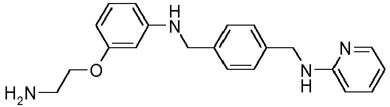

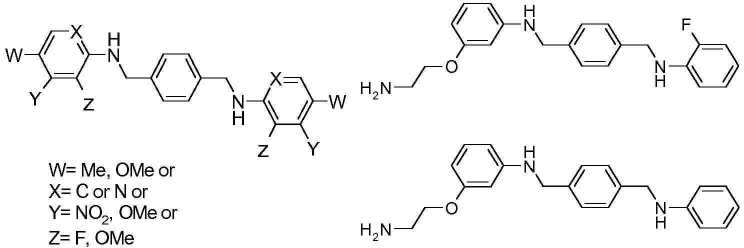

According to advantageous embodiments, the biovector is a folate-receptor-targeting biovector, such as a folic acid or any known derivative of folic acid, and in particular a derivative having the formula in document WO2004112839
(E1): 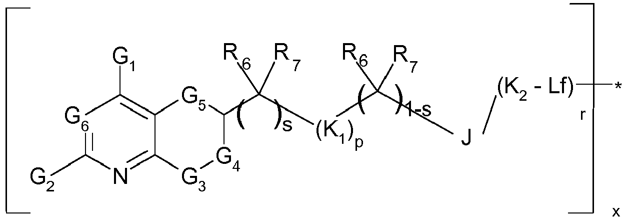
or (E2): 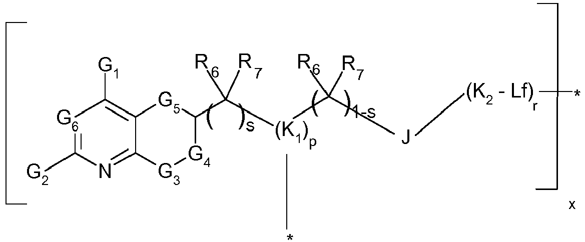 in which:
   * represents the site where the biovector is linked to the chelate;
      a) G1 is chosen independently from the group constituted of: halo, R_{f}2, OR_{f}2, SR_{f}3 and NR_{f}4R_{f}5; preferably, G1 is chosen from NH2 or OH;
      b) G2 is chosen independently from the group constituted of: halo, R_{f}2, OR_{f}2, SR_{f}3 and NR_{f}4R_{f}5;
      c) G3, G4 represent divalent groups chosen independently from the group constituted of -(R_{f}6')C=,-N=,-(R_{f}6')C(R_{f}7')-, -N(R_{f}4')-;
         preferably, G3 is -N=(folic acid) or -CH- (compounds described hereafter: CB3717, raltitrexed, MAI) when the ring comprising G3 is aromatic, and G3 is -NH- or -CH₂- (compounds described hereafter: AG-2034, lometrexol) when the ring comprising G3 is nonaromatic;
         preferably, G4 is -CH- or -C(CH₃)- when the ring comprising G3 is aromatic, and -CH₂- or -CH(CH₃)- when the ring comprising G3 is nonaromatic;
      e) G5 is absent (pemetrexed compound) or chosen from -(R_{f}6')C=,-N=, -(R_{f}6')C(R_{f}7')-, -N(R_{f}4')-;
      f) J is a 5- or 6-membered heterocyclic or nonheterocyclic aromatic ring, it being possible for the atoms of the ring to be C, N, O, S;
      g) G6 is N or C (compound described hereafter: 3-deaza-ICI-198,583);
      h) K1 and K2 are chosen independently from the group constituted of -C(Z_{f})-, -C(Z_{f})O-, -OC(_{Zf})-, -N(R_{f}4")-, -C(Z_{f})-N(R_{f}4), -N(R_{f}4")-C(Z_{f}), -O-C(Z_{f})-N(R_{f}4")-, -N(R_{f}4")-C(Z_{f})-O-, N(R_{f}4")-C(Z_{f})-N(R_{f}5")-, -O-, -S-, -S(O)-,-S(O)₂-, -N(R_{f}4")S(O)₂-, -C(R_{f}6")(R_{f}7")-, -N(C ≡ CH)-, -N(CH₂-C ≡ CH)-, C₁-C₁₂ alkyl and C₁-C₁₂ alkoxy; in which Zf is O or S; preferably, K1 is -N(R_{f}4")- or -C(R_{f}6")(R_{f}7")- with R_{f}4", R_{f}6" and R_{f}7" being H; A2 optionally being covalently bonded to an amino acid;
      i) R_{f}2, R_{f}3, R_{f}4, R_{f}4', E_{f}4", R_{f}5, R_{f}5"', R_{f}6" and R_{f}7" are chosen independently from the group constituted of: H, halo, C₁-C₁₂ alkyl, C₁-C₁₂ alkoxy, C₁-C₁₂ alkanoyl, C₂-C₁₂ alkenyl, C₂-C₁₂ alcynyl, (C₁-C₁₂ alkoxy)carbonyl, and (C,-C, 2 alkylamino) carbonyl;
      h) R_{f}6' and R_{f}7' are chosen independently from the group constituted of: H, halo, C₁-C₁₂ alkyl and C₁-C₁₂ alkoxy; or R_{f}6' and R_{f}7' together form O=;
      i) Lf is a divalent linker which includes, where appropriate, a natural amino acid or a natural polyamino acid, linked to K2 or to K1 via its alpha-amino group, via an amide bond;
      j) p, r and s are independently 0 or 1;
      k) x is an integer between 1 and 5, advantageously equal to 1.

The formula (E) includes the tautomeric forms, for example compounds in which G1 is OH, SH or NH.

For the compounds of the invention in which at least one of the groups K1, K2,
R_{f}1, R_{f}2, R_{f}3, R_{f}4, R_{f}4', R_{f}4", R_{f}5, R_{f}5", R_{f}6, R_{f}7", R_{f}6, R_{f}7, R_{f}6' and R_{f}7' comprises an alkyl, alkoxy, alkylamino, alkanoyl, alkenyl, alkynyl, alkoxycarbonyl or alkylaminocarbonyl group, the group preferably contains 1 to 6 carbon atoms (C₁-C₆), more preferably 1 to 4 carbon atoms (C₁-C₄).

Among the compounds stated above, the inventors have in particular focused on the derivatives
a)

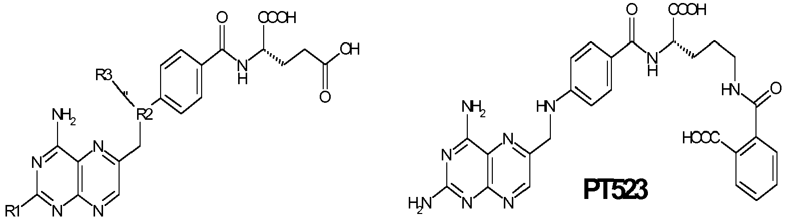

| | R1 | R2 | R3 | | | R1 | R2 | R3 |
|---|---|---|---|---|---|---|---|---|
| MTX | NH₂ | N | CH₃ | | 2-dAMT | H | N | H |
| 2-dMTX | H | N | CH₃ | | 2-CH₃-AMT | CH₃ | N | H |
| 2-CH₃-MTX | CH₃ | N | CH₃ | | Edatrexate | NH₂ | c | C₂H₅ |
| AMT | NH₂ | N | H | | | | | |

b)

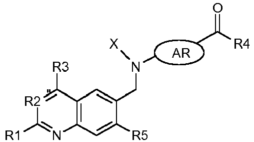

| X = propargyl | R1 | | R3 | R4 | R5 |
|---|---|---|---|---|---|
| CB3717 | NH₂ | N | OH | Glu | H |
| ICI-198,583 | CH₃ | N | OH | Glu | H |
| 3-deaza-ICI-198,583 | CH₃ | CH | OH | Glu | H |
| 4-H-ICI-198,583 | CH₃ | N | H | Glu | H |
| 4-OCH₃-ICI-198,583 | CH₃ | N | OCH₃ | Glu | H |
| Glu→Val-ICI-198,583 | CH₃ | N | OH | Valine | H |
| Glu→Sub-ICI-198,583 | CH₃ | N | OH | Suberate | H |
| 7-CH₃- ICI-198,583 | CH₃ | N | OH | Glu | CH₃ |
| X = methyl | R1 | R2 | R3 | R4 | R5 |
| Raltitrexed | CH₃ | N | OH | Glu | H |
| 2-NH₂-ZD1694 | NH₂ | N | OH | Glu | H |

Thus, advantageously, the biovector is a folate or a folate derivative.

The folate derivatives thus include the following compounds: pteropolyglutamic acid, pteridines capable of targeting the folate receptor (tetrahydropterins, tetrahydrofolates, dihydrofolates in particular).

The folate derivatives also include the following compounds: aminopterin, amethopterin (methotrexate), N-methylfolate, 2-deaminohydroxyfolate; and deaza derivatives thereof such as 1-deazamethopterin or 3-deazamethopterin, and 3',5'-dichloro-4-amino-4-deoxy-N-methylpteroylglutamic acid (dichloromethotrexate).

The folate derivatives include the deaza or dideaza compounds. The terms "deaza" and "dideaza" refer to the known derivatives, which do not have nitrogen atoms G3, G5, G6 of folic acid. For example, the deaza derivatives include the 1-deaza, 3-deaza, 5-deaza, 8-deaza and 10-deaza derivatives. Patents WO2007042504 and WO2004112839 (incorporated by way of reference) by the applicant illustrate numerous examples of chemical coupling between various biovectors and various chelates, typically by means of chemical linkers. By way of examples, mention will be made of the following linkers:
1) amino acids.
2) linkers L capable of interacting with at least one biovector functional group and at least one chelate functional group. L includes in particular alkyl chains which are substituted or unsubstituted, saturated or unsaturated, and straight or branched, peptides, polyethylene glycols and polyoxyethylenes. Mention will in particular be made of:
   3) a.1) (CH₂)₂-phenyl-NH, (CH₂)₃-NH, NH-(CH₂)₂-NH, NH-(CH₂)₃-NH, nothing or a single bond, (CH₂)ₙ, (CH₂)ₙ CO-, -(CH₂)ₙNH-CO- with n = 2 to 10, (CH₂CH₂O)_{q}(CH₂)ᵣ-CO-, (CH₂CH₂O)_{q}(CH₂)ᵣ-NH-CO- with q = 1-10 and r=2-10, (CH₂)ₙ-CONH-, (CH₂)ₙ-CONH-PEG, (CH₂)ₙ-NH-, 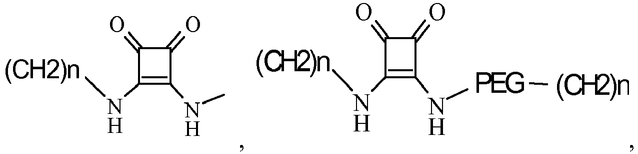 with n=1 to 5 and advantageously n=4 or 5, HOOC-CH₂-O-(CH₂)₂-O-(CH₂)₂-O-CH₂-COOH; HOOC-(CH₂)₂-CO₂-(CH₂)₂-OCO-(CH₂)₂-COOH; HOOC-CH(OH)-CH(OH)-COOH; HOOC-(CH₂)ₙ-COOH; NH₂-(CH₂)ₙ-NH₂, with n=0-20; NH₂-(CH₂)ₙ-CO₂H; NH₂-CH₂-(CH₂-O-CH₂)ₙ-CO₂H with n=1 to 10, linkers denoted A8 to A32 of document WO 2006/095234, pages 104 and 105.
      a.2) P1-1-P2, which may be identical or different, P1 and P2 being chosen from O, S, NH, nothing, CO₂, NHCO, CONH, NHCONH, NHCSNH, SO₂NH-, NHSO₂- and squarate
         with 1= alkyl, alkoxyalkyl, polyalkoxyalkyl (PEG), alkyl interrupted with one or more squarates or with one or more aryls, advantageously phenyls, alkenyl, alkynyl, alkyl which are interrupted with one or more groups chosen from - NH-, -O-, -CO-, -NH(CO)-, -(CO)NH-, -O(CO)-, or -(OC)O-
         L will, for example, have a molecular weight of between 300 and 2000 g/mol, in particular between 300 and 1000 g/mol.
         P1 and P2 are thus groups for coupling the linker with, on the one hand, the chelate and, on the other hand, the biovector.
   4) linkers described in patent US 6 264 914, capable of reacting with amino, hydroxyl, sulfhydryl, carboxyl, carbonyl, carbohydrate, thioether, 2-aminoalcohol, 2-aminothiol, guanidinyl, imidazolyl and phenolic functional groups (of the biovector and of the chelate); and with the definition summaries of WO 2007/042504.
   5) certain linkers described in patent US 6 537 520 of formula (Cr₆r₇)_{g}-(W)ₕ-(Cr₆ₐr₇ₐ)_{g'}-(Z)ₖ-(W)_{h'}-(Cr₈r₉)g"-(W)_{h"}-(Cr₈ₐr₉ₐ)_{g'"} with the definitions of this document.
   6) certain linkers described in document WO 02/085908, for example a linear or branched chain of a linker, chosen from:
      - CR6"'R7"'-, - (R6"')C=C(R7"')=, -CC-, -C(O)-, -O-, -S-, -SO₂-, -N(R3"')-,-(R6"')C=N-, -C(S)-, -P(OO(OR3'"))-, -P(O)-(OR3"')O-, with R"'3 being a group capable of reacting with a nitrogen or an oxygen,
      - a cyclic region (divalent cycloalkyls, divalent heterocycles)
      - polyalkylenes, polyalkylene glycols.
   7) linkers of document WO 03/011115 pages 124-125, in particular

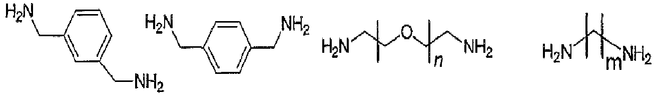

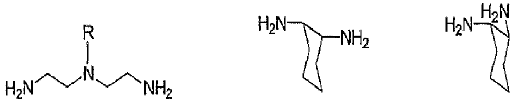

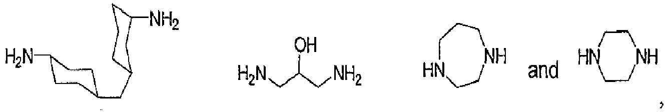

The choice of the linkers (structure and size) may be made in particular in such a way as to control in particular the charge, the lipophilicity and/or the hydrophilicity of the product as a function of the desired diagnostic indication, so as to optimize the biological targeting and/or the biodistribution. Linkers which are biodegradable in vivo, PEG linkers or mini-PEG linkers may in particular be used.

By way of examples, the products vectorized and radiolabeled, and solubilized in a buffer solution using the buffers of the applicant (lactate, tartrate, malate in particular) form gallium metal complexes of formula: 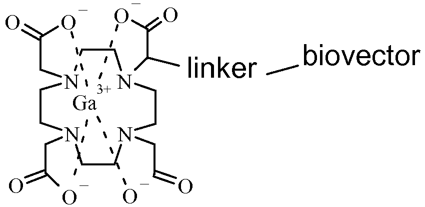 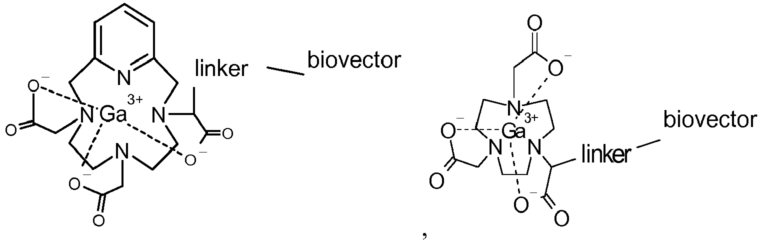 with the previous definitions of the terms linkers and biovectors indicated above.

The buffers used are typically under the form of a buffer solution comprising at least a conter-ion, in particular sodium or ammoniumor an ion of an organic base (advantageously an organic polybase such as meglumine, ethylenediamine, diethylenetriamine, tetramethylethylenediamine, triethylenetetramine). For illustration, the lactate buffer is used as sodium lactate or ammonium lactate, for which the applicant has shown the efficiency.

The invention also relates to a method for preparing a radiolabeled contrast product, comprising the following steps:
- preparing a solution of radionuclide, gallium 68
- preparing a vectorized NOTA or PCTA chelate,
- mixing, in a buffer solution according to the present invention, at ambient temperature without heating and advantageously without microwaves, in less than 15 minutes, preferably in less than 10 minutes, more preferably in less than 7 minutes, the solution of radionuclide and of the vectorized chelate.

Advantageously, the buffer is chosen from: lactate, tartrate, malate, succinate, maleate, ascorbate, carbonate and phosphate buffers, and mixtures thereof, even more advantageously from lactate, tartrate and carbonate buffers, and mixtures thereof.

Those skilled in the art understand that, in customary practice, different variants are possible for this mixture. Typically, the vectorized chelate (not yet radio labelled) is provided in the form of a concentrated aqueous solution, before the introduction of the buffer solution, and the acid eluate (pH around 2) of Ga68. For example, as illustrated hereafter, a volume of 0.4 ml of 0.6 nM Ga68 solution is added to the reactor which contains 1 ml of buffer solution and 20 µl of 1.2 M vectorized chelate solution. However, it is understood that all the variants which give an equivalent result for the complexation are included in the present invention. For example, a greater amount of Ga68 solution at an appropriate dilution may be provided. For example, for reasons of biovector storage, the labeled chelate is provided in buffer solution and not in an aqueous solution different than the buffer.

The invention also describes the various intermediate products where appropriate required for the complexation in the buffer solutions of the invention, and in particular:
- an eluate of Ga68 (the eluate being produced by a Ga68 generator) in solution in 98% of acetone, 0.05M Hcl
- a buffer solution chosen from the buffers of the present application, advantageously lactic, tartaric, succinic, ascorbic, phosphoric, and carbonic buffers, and combinations thereof;
- a vectorized NOTA or PCTA chelate, not yet complexed with the Ga68 radionuclide, in buffer solution or in water, the buffer being chosen from the buffers of the present application, advantageously lactic, tartaric, succinic, ascorbic, phosphoric and carbonic buffers, and combinations thereof.

A combination of buffers can also be used as complexation buffer solution, in particular a combination of at least two buffers mentioned above, for example a mixed lactic-succinic or lactic-tartaric buffer, in proportions of advantageously 90/10, 80/20, 70/30, 60/40, 50/50 for combinations of two buffers.

According to another aspect, the invention relates to an injectable solution comprising a buffer and a chelate selected from NOTA, PCTA, vectorised NOTA or vectorised PCTA, complexed with a radionuclide, ⁶⁸Ga, the buffer comprising between two and five functions for coordination with the radionuclide, each coordination function being independently chosen from a carboxylic acid function and a hydroxyl function, on the condition that the buffer comprises at least one carboxylic acid function and at most two carboxylic acid functions. The buffer is a mono-, di-, tri- or tetrahydroxylated C₁-C₁₀ monocarboxylic acid, or a C₁-C₁₀ dicarboxylic acid which is optionally mono-, di-, or trihydroxylated and optionally unsaturated.

The characteristics of the buffers and of the chelates are as defined above. Advantageously, the buffer solution is a solution at from 0.05 to 1.5 M with respect to buffer, preferably from 0.1M to 1M with respect to buffer ; advantageously 0.1 to 0.5 M, for instance 0.1 to 0.3 M.

Advantageously, the concentration of the radiolabeled vectorized chelate in the injectable buffer solution is between 0.1 and 100 µM, for example 0.5 to 20 µM, in particular 1 to 10 µM.

Advantageously, the solution administered to the patient also comprises at least one excipient that is suitable for limiting radiolysis, i.e. decomposition of the diagnostic compound administered. This is advantageously in particular when a diagnostic solution of multidose/multipatient type, which involves being able to successfully store the product, is prepared. Antiradiolysis agents are known to those skilled in the art and summarized in particular in WO2007042504 and WO2005009393 (examples: free-radical blockers, dithiocarbamates, PDTC, soluble selenium compounds such as selenomethionine or selenocystine with, where appropriate, sodium ascorbate, derivatives capable of reducing oxidized amino acids, such as methionine, in particular thiol derivatives of cysteine, mercaptoethanol, dithiothreitol). Arginine or lysine formulations may also be used.

The applicant's products in buffer solution have the great advantage that they can be used without heating, but may, where appropriate, if the radiopharmacy is not from time to time equipped with a complexation instrument without a heating device, be used in an instrument with a heating device. Various automic complexationinstruments may be used, for instance equiped with known either cationic or anionic resins.

The present description also describes an instrument (complexation system), which is preferably automatic, without a heating device, comprising at least one compartment for mixing a solution of radionuclide provided by a radionuclide generator, more especially a Ga68 generator, a solution of noncomplexed vectorized chelate and a buffer solution, said buffer being chosen from lactate, tartrate, maleate, succinate, malate, ascorbate, carbonate and phosphate and mixtures thereof.

Any suitable device is prepared accordingly. For example, the Ga68 provided is a sterile solution separated into single doses by the automated machine by means of a distribution device of the automated machine, or before the automated machine, with the use of single-dose containers introduced into the automated machine. The automated machine comprises, for example, a mixing compartment capable of preparing several doses of injectable radiolabeled product for one or more patients. The automated machine may also comprise several mixing subcompartments, each compartment being able to mix a dose of Ga68 radionuclide in particular and a dose of vectorized product. The automated machine may be automatic.

The automated machine, which where appropriate is programmable, may also prepare several different injectable products, with identical or different biovectors, and different doses and concentrations, where appropriate as a function of the type of product to be produced and of the method of injection (manual or automatic injection). The various products, methods and instruments of the application may in addition be used for any appropriate imaging modalities, in particular PET imaging, multimodal imaging and, where appropriate, product multi-injections, in particular PET/MRI, PET/scan, PET/scan/MRI and optical imaging, with the analytical and image-processing methods. The invention thus relates to any medical imaging plant comprising a complexation instrument as described in the application, in combination with MRI and/or XR-scan and/or optical equipment. More as a whole, any equipment of the prior art (summarized in particular in WO2007/042504) suitable for the complexation compounds and conditions of the invention may be used.

The detailed examples which follow :
- demonstrate the effectiveness of the applicant's buffers (example 1)
- illustrate preparation of vectorised chelates (chelate + biovector) with NOTA and PCTA chelates, and folate and peptides biovectors (it is emphasized that the one skilled in the art can thus prepare similar compounds with many other biovectors, many other linkers, many other chelates)

### Example 1: compared complexation with the buffers

The buffers are prepared at pH4. They are obtained by mixing the weak acid at 0.1M and the weak base at 0.1M.

Procedure is detailed for NOTA and PCTA chelates for illustration (similar protocols are used with other chelates) :
50 mg of NOTA or of PCTA (0.16 mmol) are dissolved in 5 ml of 0.1M buffer. 0.5 ml of a 0.5M solution of ⁶⁹GaCl₃ (1.5 eq) is added. The reaction medium is stirred at ambient temperature. Samples are taken regularly for 15 minutes and are analyzed by LC/MS.

In the following table, "complete complexation" means that the chelate has successfully complexed the gallium.

Positives results are obtained with sodium and ammonium buffers.

For citric buffer (prior art reference), the complexation is not efficient since there is only a small amount of complex (gallium complexed by chelate) whereas there is a large amount of ligand (the chelate not complexing the gallium). Examples are presented as an illustration for NOTA and PCTA :

**For NOTA:**

| **Buffer** | **Results** |
|---|---|
| Lactic | Complete complexation (single peak in HPLC) in < 15min at AT |
| Tartaric | Complete complexation (single peak in HPLC) in < 15min at AT |
| Carbonic | Complete complexation (single peak in HPLC) in < 15min at AT |
| Phosphoric | Complete complexation (single peak in HPLC) in < 15min at AT |
| Ascorbic | Complete complexation (absence of ligand) < 15min at AT |
| Succinic | Complete complexation < 15min at AT |
| Maleic | Complete complexation < 15min at AT |
| *Citric (negative results)* | *A large amount of ligand, a small amount of complex* |

**For PCTA:**

| **Buffer** | **Results** |
|---|---|
| Lactic | Complete complexation (single peak in HPLC) in < 15min at ambient temperature (AT) |
| Carbonic | Complete complexation (single peak in HPLC) in < 15min at AT |
| Ascorbic | Complete complexation (absence of ligand) < 15min at AT |
| *Citric (Negative results)* | *A large amount of ligand; a small amount of complex* |

### Example 2: Synthesis of Dideaza-NOTA:

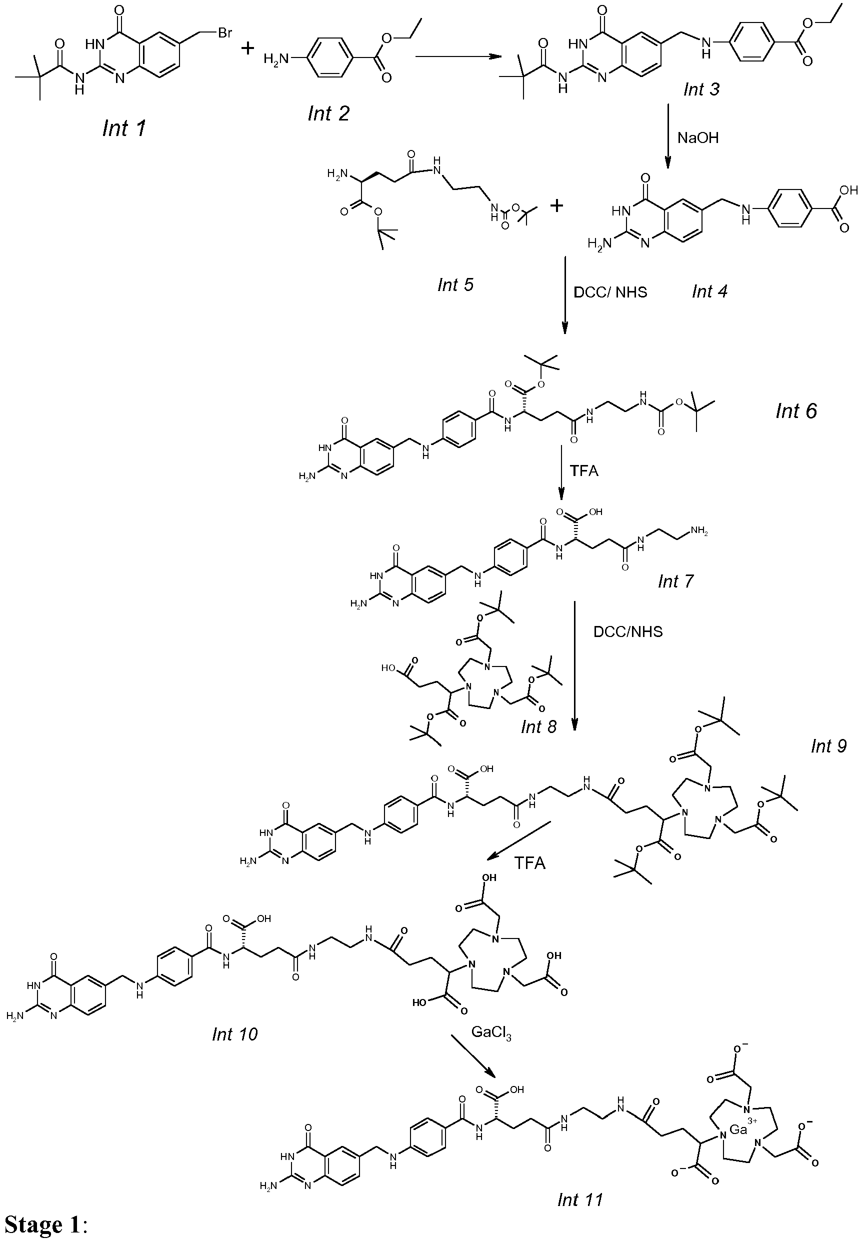

0.5 g of intermediate 2 is dissolved in 20 ml of CH₃CN, in the presence of 0.6 g of K₂CO₃. A suspension of the brominated derivative (int.1) in 20 ml of CH₃CN is added thereto. The reaction medium is maintained at reflux, under argon, with vigorous magnetic stirring, for 18 H. After a return to ambient temperature, the reaction medium is filtered. The insoluble material is taken up in 20 ml of water and then filtered. The filtrate is evaporated under pressure. The residue obtained is taken up in Et₂O and is then filtered. 1.2 g of product are obtained. [M+H]+ = 423.16

### Stage 2:

0.6 g of the intermediate obtained in the previous stage is suspended in 2.4 ml of ethanol. Dissolution is complete after the addition of 6 ml of 1M NaOH. The reaction medium is stirred for 1H30 at 70°C. After a return to ambient temperature, the medium is brought to pH 1 by adding HCl 6N. The suspension obtained is filtered and then washed thoroughly with water and subsequently with ethanol. After drying, 0.35 g of product is obtained (yield = 80%). [M+H]⁺ = 311.10

### Stage 3:

Int.4 (1.8 mmol) and Int.5 (1.8 mmol) are dissolved in DMF, at ambient temperature, under dry conditions (CaCl₂ track). 1.4 eq of HOBT (2.5 mmol) and then 1.4 eq of EDCI (2.5 mmol) are added to the reaction medium. After reaction overnight at ambient temperature, the reaction medium is precipitated from 250 ml of water. After filtration, the residue is washed with water and then dried under vacuum; 0.85 g of yellow crystals is obtained with a yield of 81%.[M+H] + = 638.30

### Stage 4:

0.266 mmol of intermediate obtained in the previous stage is dissolved in 1.8 ml of TFA. The reaction medium is left at ambient temperature for 1H and is then evaporated. The product is obtained by crystallization from 25 ml of Et₂O. 180 mg of yellow crystals are obtained, which are purified in an open column on RP2 silica, elution being carried out with water (TFA 0.05%) / CH₃CN. After freeze-drying, 50 mg of white product are obtained (yield 31%).BP: [M+H] ⁺= 482.22, [M+2H] ²⁺= 241.68

### Stage 5

### Formation of the activated ester:

After 75 mg of NOTAGA(tBu)₃ have been dissolved in 2 ml of CH₂Cl₂, 15 mg (1 eq) of NHS and then 28 mg (1 eq) of DCC are added. After reaction for half an hour at ambient temperature, the DCU formed is filtered through Whatman paper and the filtrate is concentrated to a final volume of approximately 0.5 ml.

### Amidation:

50 mg of intermediate obtained in the previous stage are dissolved in 2.5 ml of DMSO in the presence of 2 eq of NEt₃ (40µl). The activated ester, in solution in CH₂Cl₂ is added thereto. After reaction for 1H, the reaction medium is precipitated from 25 ml of Et₂O. The product obtained is used in the purification by flash chromatography (Merck SVF D26-RP18 25-40 µm-31 g silica cartridge), after having been solubilized in 50/50 (aqueous eluant phase (TFA pH2.8) /CH₃CN). After freeze-drying, 24 mg of white crystals are obtained (yield 28.4%).BP: [M+H] ⁺= 1007.5, [M+2H]²⁺ = 504.44

### Stage 6

24 mg of intermediate obtained in the previous stage are solubilized in 1 ml of TFA. After reaction for 6h at ambient temperature, the reaction medium is evaporated and taken up in 25 ml of Et₂O. 10 mg of crystals are obtained.

### Stage 7

20µl of a 1mg/ml aqueous solution of the compound obtained at the step 6 and 1 ml of sodium lactate buffer 0.5M pH3.9 are introduced in the reaction vial. Then 400µl of a solution of ⁶⁸GaCl₃ in a mixture HC10,05M/acetone are added. The reaction mixture is incubated at room temperature for 15min. The reaction vial is washed with 6 ml of water and the reaction mixture is transferred into the C-18 column which was preconditioned with 1 ml ethanol and 1 ml ultra-pure water. The final product is eluted from the cartbridge with 1 ml of a 50% ethanol/water solution.

The final product is passed through a 0.22µm Millipore filter and is diluted with NaCl 0.9% up to 6 ml.

Similar procedure is used for lactate, succinate, phosphate, maleate, malate buffers.

### Example 3 : Synthesis of folate-NOTA:

a) Compound of formula : 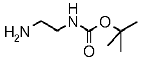
   20 g (91.7 mmol) of Boc₂O are dissolved in 40ml of CH₂Cl₂. Then a solution of 22 g (366.6 mmol) of diaminoethane in 200ml of CH₂Cl₂ is added dropwise. Le reaction vial is mixed at room temperature for 2 hours. First, the product is purified by extraction with water. The organic layer is dried over Na₂SO₄ and filtered. Then it is purified by flash chromatography on silica with a gradient of CH₂Cl₂/Methanol. 4g of a yellow oil are obtained. m/z = 161 (ES+)
b) Compound of formula : 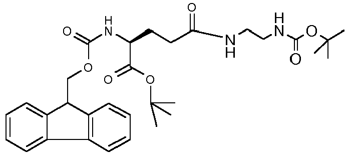
   10.27g (24 mmol) of Fmoc-Glu-OtBu are dissolved in 300 ml of CH₂Cl₂.2,8g of NHS and 4,98g de DCC are introduced. After 45minutes, the reaction mixture is filtered and added dropwise in a solution of 3.869g of the product obtained in a) dissolved in 50ml of CH₂Cl₂. After 2 hours at room temperature the product is first purified by extraction with water. The organic layer is dried over Na₂SO₄ and filtered. Then it is purified by flash chromatography on silica with a gradient of CH₂Cl₂/acetone. 7g of product are obtained. m/z = 568 (ES+)
c) Compound of formula : 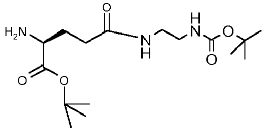
   6.5g (11.4 mmol) of the product obtained in b) are dissolved in 91ml of acetonitrile. A solution obtained with 19.5 ml of piperidine and 78 ml of acetonitrile) is added dropwise. After 2 hours at room temperature under argon atmosphere the reaction mixture is evaporated and purified by flash chromatography on silica with a gradient of CH₂Cl₂/methanol. 3.65g of oil are obtained. m/z = 346 (ES+)
d) Compound of formula : 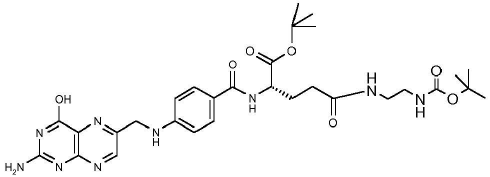
   3.3g (10.5 mmol) of pteroïc acid and 3.65g of the product obtained in c) are dissolved in 335 ml of DMSO under argon. 3.038g of EDCI and 1g of HOBT are added. The reaction mixture is heated to 40°C overnight then precipitated in water. The residue is filtered and washed with first water then Et₂O. 6g of red powder are obtained.
   m/z = 640 (ES+)
e) Compound of formula : 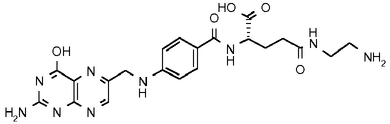
   6 g (9.3 mmol) of the product obtained in d) are dissolved in 74 ml of TFA. After 1 hour at room temperature the reaction mixture is precipitated in 800 ml of Et₂O. After filtration, 4.5g of a yellow powder are obtained.m/z = 484 (ES+)
f) Compound of formula : 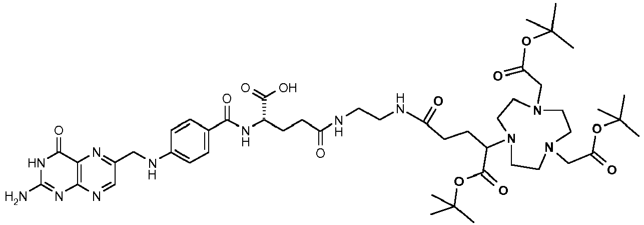
   Applying the same procedure as that described at the step 5 of the example 2 starting from:
   - 85mg of the compound obtained in e) and
   - 75mg of NOTAGA(tBu)₃
   15mg of the compound are obtained.m/z (ES+) = 1009
g) Compound of formula : 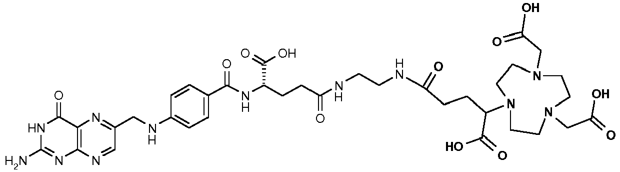
   Applying the same procedure as that described at the step 6 of the example 2 starting from 20 mg of the compound obtained in f)
   4mg of the compound are obtained.m/z (ES+) = 841
h) Compound of formula : 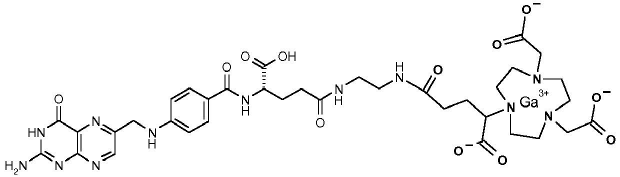
   Applying the same procedure as that described at the step 7 of the example 2

### Example 4: Synthesis of PCTA-folate (PEG linker):

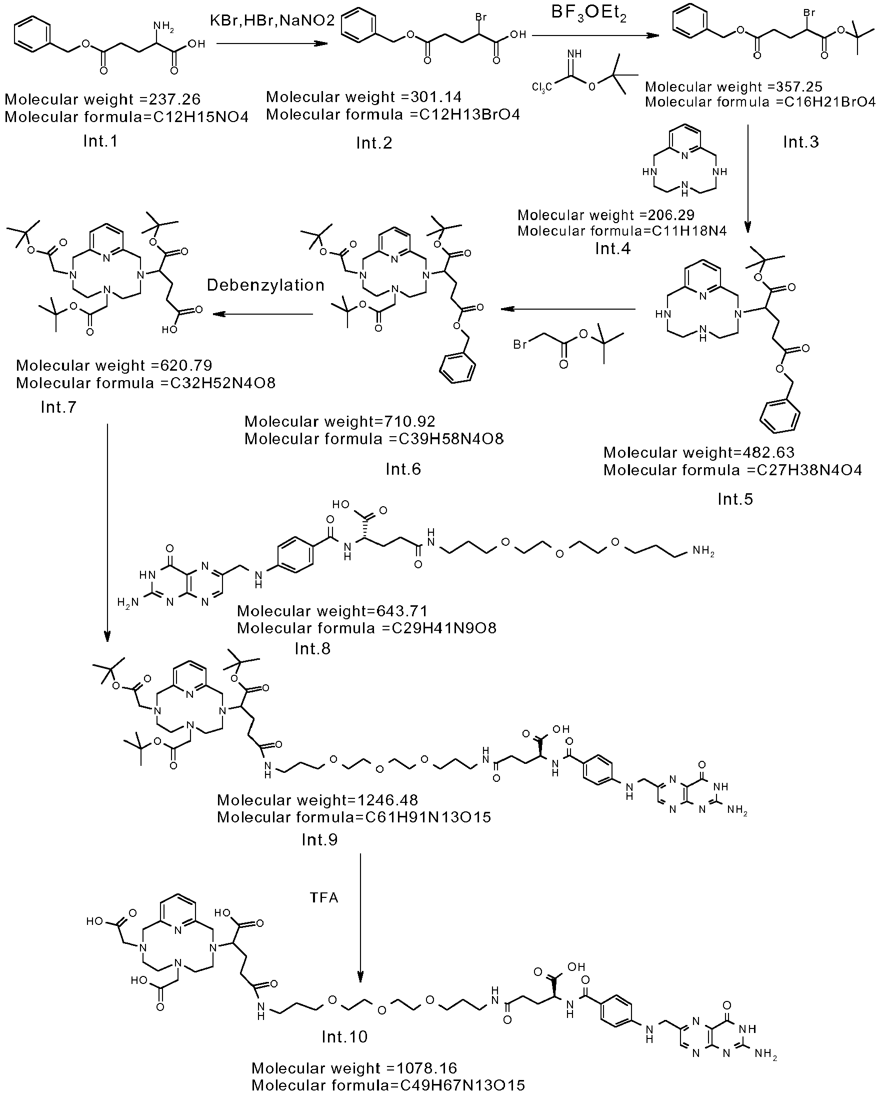

The first two stages are described in reference Bioorganic & Medicinal Chemistry Letters 10 (2000) 2133-2135.

**Step 3 :** 1.77g of Py-cyclen and 381mg of N⁺But₄Br⁻ are dissolved in 8ml of water. Then 1.63ml of triethylamine and 3g of the compound obtained at the step2 in 4ml of acetonitrile are added. Le reaction mixture is heated to 50°C
for 36 hours. After evaporation, 50ml of Et₂O are introduced and the solution is filtered. The residue is dissolved in CH₂Cl₂ and the product is purified by extraction with water. The organic layer is dried over Na₂SO₄, filtered and evaporated. 1.7g of oil are obtained. m/z = 483,31 (ES+)

### Step 4 :

700 mg of the compound obtained at the step 3 and 650 mg of K₂CO₃ are dissolved in 10 ml of acetonitrile.

A solution composed of 450 mg of ethyl bromoacetate in 15ml of acetonitrile is added and then the mixture is stirred at 70°C for 30minutes then at 50°C for 2 hours. After filtration and evaporation, the product is taken up in 80 ml of dichloromethane. The organic layer is dried over Na₂SO₄, filtered and evaporated. 0.77g of oil are obtained..m/z (ES+) = 711.36

### Step 5 :

400mg of the compound obtained at the step 4 are dissolved in 30 ml of ethanol. Palladium is added and the suspension is stirred under H₂ atmosphere for 4 hours at room temperature. After filtration and evaporation, 0.3g of oil is obtained. The product is purified by flash chromatography on C18 modified silica (25 à 40 µm Merck GX024090320LK) with a gradient of 0.05% HCOOH 0 aqueous solution and acetonitrile. 0.15g of oil is obtained. m/z (ES+) = 621.65

### Step 6 :

Applying the same procedure as that described at the step 5 of the example 2 starting from:
- 300mg of the compound obtained at the step 5 of this patent and
- 366mg of the compound obtained in e) example 11 of the patent PCT/FR/01520,
50mg of the compound are obtained.m/z (ES+) = 1246.88

### Step 7 :

Applying the same procedure as that described at the step 6 of the example 2 starting from 20mg of the compound obtained at the step 6
18mg of the compound are obtained.m/z (ES+) = 1078.47

### Step 8 :

Applying the same procedure as that described at the step 7 of the example 2

### Example 5 : chelates vectorized with a peptide

The peptide coupling method described in the examples of WO 2007/042504, pages 41-77 is used (and incorporated by reference) for instance. Peptidic biovectors are prepared, and thus coupled to the chelate (NOTA, PCTA, DOTA...) as known from the prior art. In particular :
1) for PCTA chelates :
   PCTA chelate (compound of example 6 step 3 of WO 2007/042504 page 52 (corresponding formula not complexed by gallium), is coupled to peptides as shown in example 7 of WO 2007/042504 pages 52-53
2) for NOTA chelates : NOTA chelate (compound of example 4 of WO 2007/042504 page 49, not complexed by gallium), is coupled to peptides as shown in example 5 of WO 2007/042504 page 50 ; the illustrative compounds of WO 2007/042504 page 50 (corresponding formula not complexed by gallium) are peptides coupled to NOTA with different squarate linkers ; being precised that many other linkers such as (CH2)n, PEG are prepared similarly.

Examples of compounds obtained [peptide (linear or cyclic) + linker + chelate] are presented in the following tables.

| PCTA | NOTA |
|---|---|
| 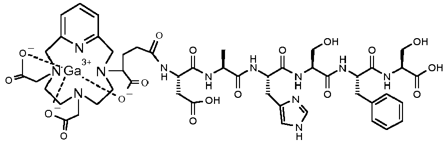 | 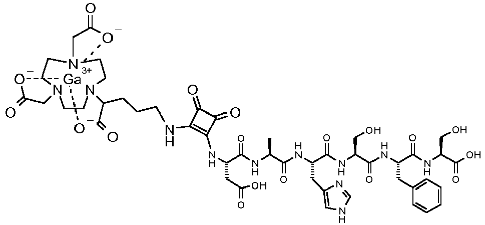 |
| 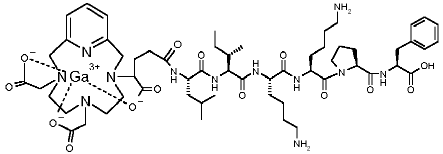 | 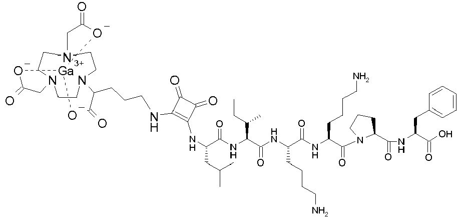 |
| 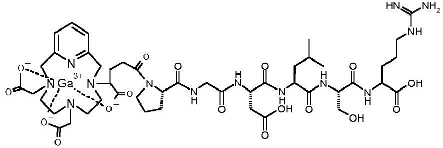 | 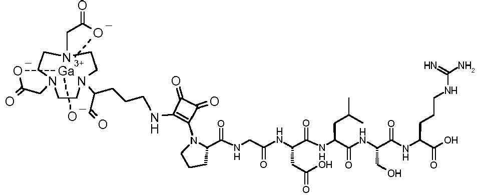 |
| 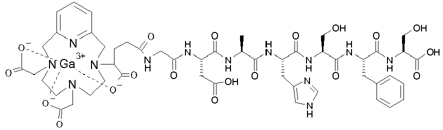 | 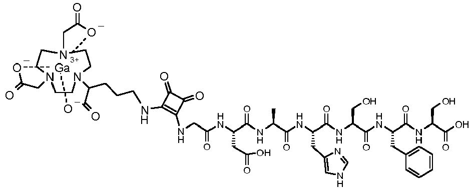 |
| 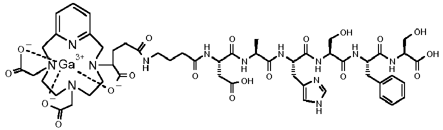 | 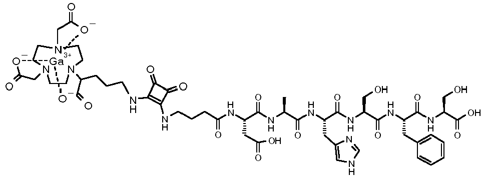 |
| 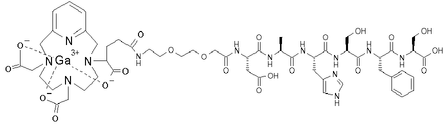 | 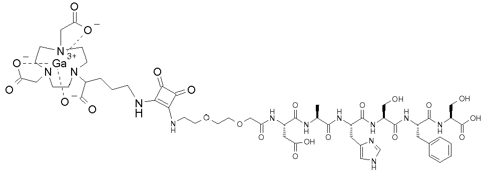 |

| DOTA |
|---|
| 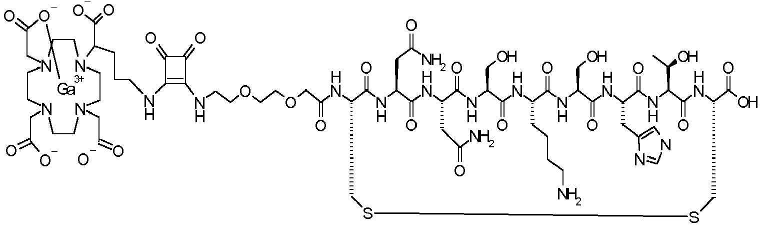 |
| 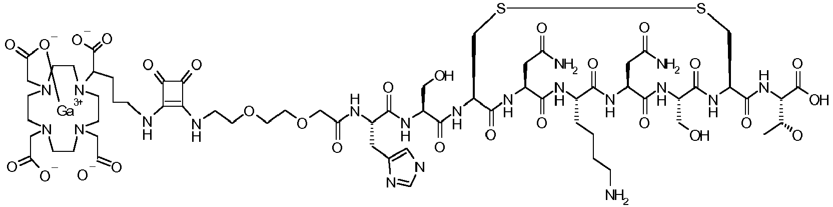 |
| 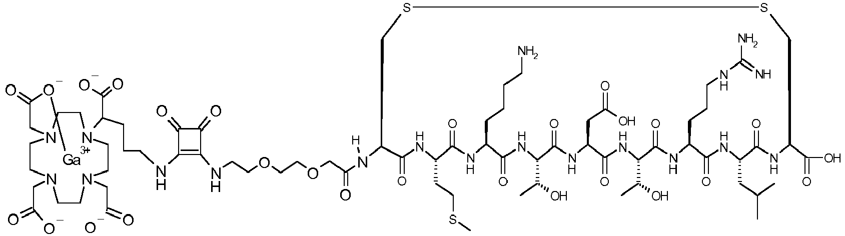 |

Appropriate protocol is advantageously used for the peptides CXCR4 or pages 23-24 of the present application (peptides a) to e)).

The compounds obtained (chelate + peptide with an eventual linker) and put into the buffer solution (lactate, tartrate, maleate, succinate ...) are thus added by the gallium solution issued from the gallium generator like in example 3 of the present application.

## Claims

1. A method for complexation of a chelate with a radionuclide, the complexation being carried out at ambient temperature without heating, by adding the radionuclide to the chelate in a buffer solution, the buffer of this solution being a mono-, di-, tri- or tetrahydroxylated C₁-C₁₀ monocarboxylic acid, or a C₁-C₁₀ dicarboxylic acid which is optionally mono-, di-, or trihydroxylated and optionally unsaturated, and the radionuclide being ⁶⁸Ga, and wherein the chelate is NOTA, PCTA, vectorised NOTA or vectorised PCTA.

2. The method as claimed in claim 1, wherein said C₁-C₁₀ monocarboxylic acid or C₁-C₁₀ dicarboxylic acid is unsaturated.

3. The method as claimed in claim 1 or 2, wherein the buffer is chosen from lactate, tartrate, malate, maleate, succinate, carbonate buffers, and mixtures thereof.

4. The method as claimed in any one of claims 1 to 3, wherein the chelate is a vectorized chelate.

5. The method as claimed in claim 4, wherein the vectorized chelate is vectorized with an agent for targeting a pathological region of diagnostic interest, chosen from an amino acid, a peptide, a polypeptide, a vitamin, a monosaccharide or polysaccharide, an antibody, a nucleic acid, a bicyclam or an aptamer.

6. The method as claimed in claim 4 or 5, wherein the vectorized chelate is dideaza-NOTA, folate NOTA, dideaza PCTA or folate PCTA.

7. An injectable solution comprising a buffer and a chelate complexed with a radionuclide, the buffer being a mono-, di-, tri- or tetrahydroxylated C₁-C₁₀ monocarboxylic acid, or a C₁-C₁₀ dicarboxylic acid which is optionally mono-, di-, or trihydroxylated and optionally unsaturated, and the radionuclide being ⁶⁸Ga, and wherein the chelate is NOTA, PCTA, vectorised NOTA or vectorised PCTA.

8. The solution as claimed in claim 7, wherein said C₁-C₁₀ monocarboxylic acid or C₁-C₁₀ dicarboxylic acid is unsaturated.

9. The solution as claimed in either one of clams 7 and 8, wherein the buffer is chosen from lactate, tartrate, malate, maleate, succinate, carbonate buffers, and mixtures thereof.

10. The solution as claimed in any one of claims 7 to 9, wherein the chelate is a vectorized chelate.

11. The solution as claimed in claim 10, wherein the vectorized chelate is vectorized with an agent for targeting a pathological region of diagnostic interest, chosen from an amino acid, a peptide, a polypeptide, a vitamin, a monosaccharide or polysaccharide, an antibody, a nucleic acid, a bicyclam or an aptamer.

12. The solution as claimed in claim 10 or 11, wherein the vectorized chelate is dideaza-NOTA, folate NOTA, dideaza PCTA or folate PCTA.

## Patentansprüche

1. Verfahren zur Komplexierung eines Chelats mit einem Radionuklid, wobei die Komplexierung bei Umgebungstemperatur ohne Erhitzen durch Zugabe des Radionuklids zu dem Chelat in einer Pufferlösung durchgeführt wird, wobei es sich bei dem Puffer dieser Lösung um eine mono-, di-, tri- oder tetra-hydroxylierte C₁-C₁₀-Monocarbonsäure oder eine C₁-C₁₀-Dicarbonsäure, die gegebenenfalls mono-, di-oder trihydroxyliert und gegebenenfalls ungesättigt ist, handelt und wobei es sich bei dem Radionuklid um ⁶⁸Ga handelt und wobei es sich bei dem Chelat um NOTA, PCTA, vektorisierte NOTA oder vektorisierte PCTA handelt.

2. Verfahren nach Anspruch 1, bei dem die C₁-C₁₀-Monocarbonsäure oder C₁-C₁₀-Dicarbonsäure ungesättigt ist.

3. Verfahren nach Anspruch 1 oder 2, bei dem der Puffer aus Lactat-, Tartrat-, Malat-, Maleat-, Succinat- und Carbonat-Puffern und Mischungen davon ausgewählt wird.

4. Verfahren nach einem der Ansprüche 1 bis 3, bei dem es sich bei dem Chelat um ein vektorisiertes Chelat handelt.

5. Verfahren nach Anspruch 4, bei dem das vektorisierte Chelat mit einem Mittel zur Abzielung auf eine pathologische Region von diagnostischem Interesse, das aus einer Aminosäure, einem Peptid, einem Polypeptid, einem Vitamin, einem Monosaccharid oder Polysaccharid, einem Antikörper, einer Nukleinsäure, einem Bicyclam oder einem Aptamer ausgewählt ist, vektorisiert ist.

6. Verfahren nach Anspruch 4 oder 5, bei dem es sich bei dem vektorisierten Chelat um Didesaza-NOTA, Folat-NOTA, Didesaza-PCTA oder Folat-PCTA handelt.

7. Injektionslösung, umfassend einen Puffer und ein mit einem Radionuklid komplexiertes Chelat, wobei es sich bei dem Puffer um eine mono-, di-, tri-oder tetrahydroxylierte C₁-C₁₀-Monocarbonsäure oder eine C₁-C₁₀-Dicarbonsäure, die gegebenenfalls mono-, di- oder trihydroxyliert und gegebenenfalls ungesättigt ist, handelt und wobei es sich bei dem Radionuklid um ⁶⁸Ga handelt und wobei es sich bei dem Chelat um NOTA, PCTA, vektorisierte NOTA oder vektorisierte PCTA handelt.

8. Lösung nach Anspruch 7, wobei die C₁-C₁₀-Mono-carbonsäure oder C₁-C₁₀-Dicarbonsäure ungesättigt ist.

9. Lösung nach einem der Ansprüche 7 und 8, wobei der Puffer aus Lactat-, Tartrat-, Malat-, Maleat-, Succinat- und Carbonat-Puffern und Mischungen davon ausgewählt ist.

10. Lösung nach einem der Ansprüche 7 bis 9, wobei es sich bei dem Chelat um ein vektorisiertes Chelat handelt.

11. Lösung nach Anspruch 10, wobei das vektorisierte Chelat mit einem Mittel zur Abzielung auf eine pathologische Region von diagnostischem Interesse, das aus einer Aminosäure, einem Peptid, einem Polypeptid, einem Vitamin, einem Monosaccharid oder Polysaccharid, einem Antikörper, einer Nukleinsäure, einem Bicyclam oder einem Aptamer ausgewählt ist, vektorisiert ist.

12. Lösung nach Anspruch 10 oder 11, wobei es sich bei dem vektorisierten Chelat um Didesaza-NOTA, Folat-NOTA, Didesaza-PCTA oder Folat-PCTA handelt.

## Revendications

1. Procédé de complexation d'un chélate avec un radionucléide, la complexation étant conduite à température ambiante sans chauffage, par ajout du radionucléide au chélate dans une solution tampon, le tampon de cette solution étant un acide monocarboxylique en C₁-C₁₀ mono-, di-, tri- ou tétrahydroxylé, ou un acide dicarboxylique en C₁-C₁₀ qui est facultativement mono-, di-, ou trihydroxylé et facultativement insaturé, et le radionucléide étant ⁶⁸Ga, et dans lequel le chélate est NOTA, PCTA, NOTA vectorisé ou PCTA vectorisé.

2. Procédé selon la revendication 1, dans lequel ledit acide monocarboxylique en C₁-C₁₀ ou acide dicarboxylique en C₁-C₁₀ est insaturé.

3. Procédé selon la revendication 1 ou 2, dans lequel le tampon est choisi parmi des tampons lactate, tartrate, malate, maléate, succinate, carbonate, et des mélanges de ceux-ci.

4. Procédé selon l'une quelconque des revendications 1 à 3, dans lequel le chélate est un chélate vectorisé.

5. Procédé selon la revendication 4, dans lequel le chélate vectorisé est vectorisé avec un agent de ciblage d'une région pathologique d'intérêt diagnostique, choisie parmi un acide aminé, un peptide, un polypeptide, une vitamine, un monosaccharide ou un polysaccharide, un anticorps, un acide nucléique, un bicyclame ou un aptamère.

6. Procédé selon la revendication 4 ou 5, dans lequel le chélate vectorisé est didésaza-NOTA, folate-NOTA, didésaza-PCTA ou folate-PCTA.

7. Solution injectable comprenant un tampon et un chélate complexé avec un radionucléide, le tampon étant un acide monocarboxylique en C₁-C₁₀ mono-, di-, tri- ou tétrahydroxylé, ou un acide dicarboxylique en C₁-C₁₀ qui est facultativement mono-, di-, ou trihydroxylé et facultativement insaturé, et le radionucléide étant ⁶⁸Ga, et dans lequel le chélate est NOTA, PCTA, NOTA vectorisé ou PCTA vectorisé.

8. Solution selon la revendication 7, dans laquelle ledit acide monocarboxylique en C₁-C₁₀ ou acide dicarboxylique en C₁-C₁₀ est insaturé.

9. Solution selon l'une quelconque des revendications 7 et 8, dans laquelle le tampon est choisi parmi les tampons lactate, tartrate, malate, maléate, succinate, carbonate, et des mélanges de ceux-ci.

10. Solution selon l'une quelconque des revendications 7 à 9, dans laquelle le chélate est un chélate vectorisé.

11. Solution selon la revendication 10, dans laquelle le chélate vectorisé est vectorisé avec un agent de ciblage d'une région pathologique d'intérêt diagnostique, choisie parmi un acide aminé, un peptide, un polypeptide, une vitamine, un monosaccharide ou un polysaccharide, un anticorps, un acide nucléique, un bicyclame ou un aptamère.

12. Solution selon la revendication 10 ou 11, dans laquelle le chélate vectorisé est didésaza-NOTA, folate-NOTA, didésaza-PCTA ou folate-PCTA.
